# EUROPEAN PATENT SPECIFICATION

(11) **EP 1 466 749 B1**
(45) Date of publication and mention of the grant of the patent: **12.05.2010**
(21) Application number: 04008502.9
(22) Date of filing: 07.04.2004
(51) Int. Cl.: B41M 5/00

(54) **Ink-absorbing recording medium and method of manufacturing the same**
Tintenabsorbierendes Aufzeichnungsmedium und Verfahren zu dessen Herstellung
Support d'enregistrement d'absorption d'encre et procédé pour sa fabrication

(30) Priority: 07.04.2003 JP 2003102736
(43) Date of publication of application: 13.10.2004
(73) Proprietor: Yuen Foong Yu Paper MFG Company, Limited, Taipei, Taiwan (TW)
(72) Inventor: Chen, Min-Kong, Taipei (TW)
(74) Representative: Epping - Hermann - Fischer

(56) References cited:
- EP-A- 0 701 904
- EP-A- 0 749 845
- EP-A- 1 048 480
- EP-A- 1 188 573
- EP-A- 1 195 259
- EP-A- 1 211 086
- EP-A- 1 277 591
- US-A- 4 636 805
- US-A- 4 801 497
- PATENT ABSTRACTS OF JAPAN vol. 017, no. 497 (M-1476), 8 September 1993 (1993-09-08) & JP 05 124330 A (CANON INC), 21 May 1993 (1993-05-21)
- PATENT ABSTRACTS OF JAPAN vol. 017, no. 151 (M-1387), 25 March 1993 (1993-03-25) & JP 04 320877 A (ASAHI GLASS CO LTD), 11 November 1992 (1992-11-11)

## Description

The present invention is related to an ink-absorbing recording medium and the manufacturing method thereof, and more particularly to a manufacturing method for a highly absorbing and unfading ink-absorbing recording medium.

In the past, the subjects about ink-absorbing recording medium used for a recording paper and the manufacturing method thereof are widely discussed. For example, the method for making a glossy ink-absorbing recording medium includes the steps of smearing polyvinyl over the two surfaces (the outside surface and the inside surface) of the base material, pressing the base material, and then smearing aluminum oxide, inorganic substance and aqueous solution mastic containing polyvinyl alcohol over the outside surface of the base material. For convenience, here we name this method as method A.

A good ink-absorbing recording medium can be manufactured by the method A. However, since ink can only be absorbed by the aluminum oxide layer, there is a need to increase the thickness of the layer. In addition, even if the ink is absorbed, the dyes could be spread easily and hence the anti-fading function has no effect because of the remained non-volatile solution.

For making ink-absorbing recoding medium with higher quality, another method was developed. The method includes the steps of smearing aqueous solution having polyvinyl alcohol and borax to protect the surface of the paper base material from being wrinkled and split after dried, applying liquid containing aluminum oxide and polyvinyl alcohol to the base material to form a membrane layer, moistening the base material after it is dried, and casting the base material by pressing it on the surface of a heated and glossy metal. For convenience, here we name this method as method B.

In comparison with method A, an ink-absorbing recording medium with higher quality can be made through method B. Through method A, the luster rate of the surface of the ink-absorbing recording medium at the angle of incidence of 20° is about 20%, while it is more than 30% through method B.

Besides, the solution for the applying process in the past is an aqueous solution by dissolving non-ionic or anionic polyvinyl alcohol and aluminum oxide in water.

However, in the past, the ink-absorbing recording medium is applied by using the above-mentioned liquids containing non-ionic or anionic polyvinyl alcohol to form a membrane layer. Unfortunately, the membrane layer has bad ink-absorbing ability and is not endurable to weather, so it is necessary to enhance these properties. Besides, it is also necessary to continue enhancing the quality of the ink-absorbing recording medium.

In the prior art document EP 1 195 259 A2, an ink jet-recording sheet for forming recorded images using liquid ink is described, wherein the ink jet-recording sheet comprises (a) at least one member with a specific compound including a sulfur atom therein, and (b) at least one member selected from a group consisting of homopolymers of diallylamine compounds and copolymers with copolymerizable ethylenically unsaturated compounds.

In the European patent application publication EP 1 188 573 A2, a recording material is provided, wherein the recording material comprises 1) a sheet-like paper substrate, 2) at least one pigment layer coated thereon, and 3) at least one sealing layer coated thereon which comprises a particulate polymer dispersion characterized by a film forming temperature between 60°C and 140°C (up to: 160°C) and an average particle size between about 1 µm and about 50 µm together with at least one water-soluble polymer.

In the prior art document EP 1 211 086 A1, an ink jet recording element is disclosed for improving the occurrence of cracks in its ink-receiving layer. The recording medium provided therein comprises a layer with a high amount of pigment and a top layer containing a water soluble polymer.

The European patent application publication EP 1 048 480 A2 discloses an ink-jet recording material having a support material and pigment layers provided thereon, wherein the pigment layers comprise at least a lower layer containing barium sulphate and an upper layer containing an aluminum oxide.

In view of previous discussion, it is an object of the present invention to provide a high-quality ink-absorbing recording medium and the manufacturing method thereof.

The main purpose of the present invention is to provide a method for manufacturing ink-absorbing recording medium. The ink-absorbing recording medium made by this method has properties of good ink-absorbing and weather-enduring abilities.

It is one object of the present invention to provide an ink-absorbing recording medium which can be used to provide high-quality printing paper. The printing paper made of the ink-absorbing recording medium is suitable for printers which utilize ink, carbon powder or dyes as the recording medium. The ink-absorbing recording medium is also suitable for photographic paper because it has a high adhesion rate for carbon powder.

According to one aspect of the present invention, a method of manufacturing an ink-absorbing recording medium includes steps of providing a liquid, which is formed by one of a cationic aluminum oxide and a dispersing solution or a colloid solution which mainly includes a cationic aluminum oxide solution for being applied on a base material to form a membrane layer; drying the membrane layer; and applying a cationic polyvinyl alcohol solution to the membrane layer, wherein the cationic polyvinyl alcohol and cationic aluminium oxide repel to each other.

Preferably, the drying step is performed by one of a drying process and a semi-drying process.

Preferably, the base material is a paper material, and the cationic polyvinyl alcohol applied membrane layer is cast through a casting process so as to manufacture a glossy ink-absorbing recording medium.

According to another aspect of the present invention, a method of manufacturing an ink-absorbing recording medium is characterized by applying a liquid, which is formed by one of a cationic aluminum oxide and a dispersing solution or a colloid solution which mainly includes a cationic aluminum oxide solution and then a cationic polyvinyl alcohol solution on a base material to form a membrane layer, wherein the cationic polyvinyl alcohol and cationic aluminium oxide repel to each other.

Preferably, the cationic aluminum oxide and the cationic aluminum oxide solution both have a concentration by weight of 70%∼95% and the corresponding cationic polyvinyl alcohol has a concentration by weight of 5%∼30%.

Preferably, the base material is a paper material, the membrane layer is dried by a drying process, and the water-applied membrane layer is cast through a casting process for manufacturing a glossy ink-absorbing recording medium.

Preferably, the drying process is one of a drying process and a semi-drying process.

According to still another aspect of the present invention, an ink-absorbing recording medium has a membrane layer, wherein the membrane layer includes a cationic aluminum oxide and a cationic polyvinyl alcohol.

The foregoing and other features and advantages of the present invention will be more clearly understood through the following descriptions with reference to the drawings, wherein:

Fig. 1 is a flow chart illustrating the method of manufacturing the ink-absorbing recording medium according to the first embodiment of the present invention;

Fig. 2 is a flow chart illustrating the method of manufacturing the ink-absorbing recording medium according to the second embodiment of the present invention; and

Fig. 3 is a diagram showing the inferential states of aluminum oxide particles and polyvinyl alcohol of the ink-absorbing recording medium according to the present invention and the prior art.

The followings are the steps of the method for manufacturing the ink-absorbing recording medium according to the present invention. First, the membrane layer smearing process is proceeded by applying the dispersing solutions or the colloidal solutions, which mainly contain cationic aluminum oxide or cationic aluminum oxide solution, to the base material. Secondly, the cationic polyvinyl alcohol solution is smeared over the dried or semi-dried membrane layer to produce the ink-absorbing recording medium. Therefore, according to the present invention, the method for manufacturing the ink-absorbing recording medium utilizes the membrane liquid which contains no polyvinyl alcohol to form the membrane layer instead of utilizing the former membrane liquid which contains non-ion or anionic polyvinyl alcohol. Thirdly, cationic polyvinyl alcohol is then applied to the membrane layer to enhance the ink-absorbing and weather-enduring abilities. More specifically, in comparison with the previous ink-absorbing recording medium, which uses non-ion or anionic polyvinyl alcohol, the present ink-absorbing recording medium, which uses cationic polyvinyl alcohol, has a better weather-enduring ability and the ink-absorbing ability is not worse. Hence, the pattern won't get worse, and the ability for showing color won't be decreased.

For the previous ink-absorbing recording medium, which uses non-ion or anionic polyvinyl alcohol, low-condensation materials are added with a great quantity so that the ink-absorbing ability is reduced. However, if the quantity of low-condensation material is too little, the membrane layer will chap. Therefore, in the past, although high-condensation non-ion or anionic polyvinyl alcohol such as JM-26 (which is made by Japan Vam & Poval) is added, the maximum weight percent can only be about 10% due to its high molecular weight.

Similarly, in the past, the membrane layers are formed by using non-ion or anionic polyvinyl alcohol such as PVA117 (which is made by Kuraray) and aluminum oxide particles. Even if the aluminum oxide particles are combined with non-ion or anionic polyvinyl alcohol such as PVA117 (which is made by Kuraray) and boehmite such as DISPERAL HP 14 (which is made by Sasol), the weight percent of the added non-ion or anionic polyvinyl alcohol can only be about 10%.

As mentioned above, although the previous ink-absorbing recording medium uses non-ion or anionic polyvinyl alcohol such as JM-26 (which is made by Japan Vam & Poval), instead of cationic polyvinyl alcohol, some problems still occur. Gelation phenomenon occurred between the cationic aluminum oxide and non-ion or anionic polyvinyl alcohol causes problem that the dispersing solution can not be spread smoothly over the membrane layer. In comparison with the solid part of the aluminum oxide, non-ion or anionic polyvinyl alcohol cannot be added up to more than 10% of weight percent. Due to the small amount addition of the non-ion or anionic polyvinyl alcohol, the weather-enduring ability is not very good and the color changes or color fades would occur easily.

Besides, if the non-ion or anionic polyvinyl alcohol is used for the membrane layer of the previous ink-absorbing recording medium, materials with high molecular weight must be added to prevent chap due to waterlessness and low absorbing ability. Compared with the ink-absorbing recording medium which uses cationic polyvinyl alcohol, its ink-absorbing ability decreases because of the high molecular weight.

According to a preferred embodiment of the present invention, cationic polyvinyl alcohol such as K210 (which is made by Gohsenol) can be added to adjust the weight percent from 15% to about 30%. The ink-absorbing ability and the definition will not degenerate and absorption due to the oxidized dye oxidized by oxidation gas will not happen. Hence, even if a plenty of cationic polyvinyl alcohol is added, it will not cause degeneracy of the ink-absorbing recording medium. Besides, the active surface of the aluminum oxide can be reduced by adding a great deal of the cationic polyvinyl alcohol.

The reason why the present invention is endurable to weather is because the aluminum oxide particles in the membrane layer are covered by the cationic polyvinyl alcohol and hence the activation of the aluminum oxide is reduced. Tiny spots for adhesion of dye and ozone are expected to be reduced.

On the other hand, in the conventional art, the polyvinyl alcohol is hard to be uniformly spread on the surface of the aluminum oxide when non-ion or anionic polyvinyl alcohol and cationic aluminum oxide are used. As a result, the dye is directly absorbed by the aluminum oxide, and hence the oxidation due to ozone occurs easily.

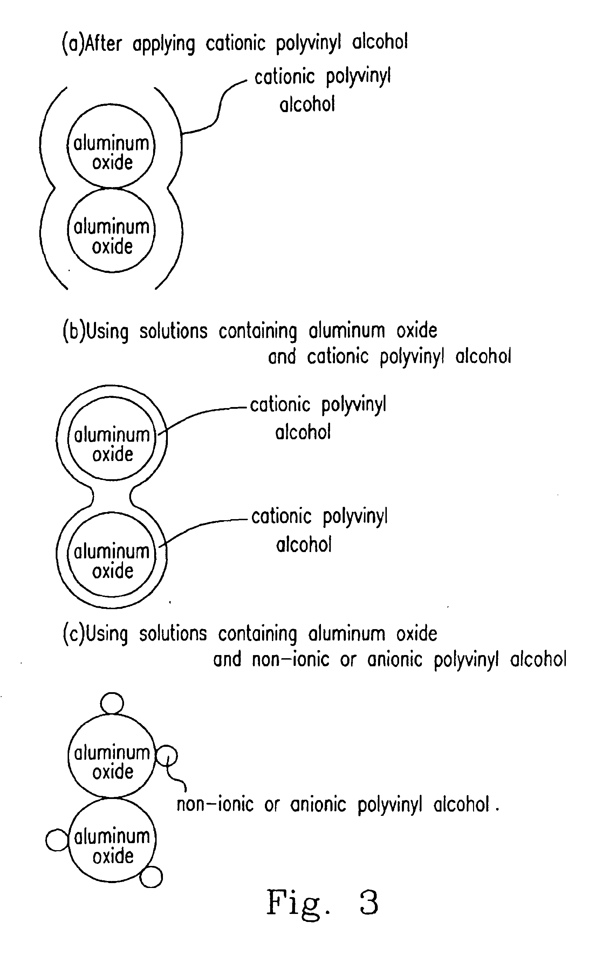

Fig. 3(a) is a diagram showing the inferential status of aluminum oxide particles and cationic polyvinyl alcohol in the membrane layer of the ink-absorbing recording medium fabricated by the above-mentioned process.

Fig. 3(c) is a diagram showing the inferential status of aluminum oxide particles and non-ionic or anionic polyvinyl alcohol in the membrane layer of the ink-absorbing recording medium fabricated by the conventional method.

As described in Fig. 3(c), when cationic aluminum oxide particles and non-ion or anionic polyvinyl alcohol are used, the gelation is not uniform, and the rate that the surfaces of aluminum oxide particles are exposed and turned into active points for ozone is very high.

On the other hand, as illustrated in Fig. 3(a), the active points can be covered by smearing cationic polyvinyl alcohol over the adjacent aluminum oxide particles. In the aspects of using oxidizing gas to protect dye from fading, antioxidant is used in the past, and unfortunately the effects are stopped on antioxidant and oxidizing gas with the same valence. However, through the present method, active points which are worsened in oxidization can be covered physically; this can prevent these active points from continuing worsened in oxidization.

Next, we are going to describe in detail the steps of smearing the liquids or colloids which contain aluminum oxide over the base material, and smearing the polyvinyl alcohol dissolved in the water over the dried or semi-dried aluminum oxide membrane layer. In the present invention, because the aluminum oxide is cationic and repels to cationic polyvinyl alcohol, the cationic polyvinyl alcohol can adhere to the membrane layer uniformly. However, in the conventional art, the gelation phenomenon on the surface of aluminum oxide would occur when non-ion or anionic polyvinyl alcohol is used. Hence, the poriferous surface formed by the aluminum oxide is covered and the ink-absorbing ability becomes so bad that it is useless.

The embodiments of the present invention are described below. First, for the base materials, if there is no casting process, all kinds of materials can be used, for example, paper base materials, aluminum oxide base materials, and paper base materials processed with laminating process. For the purposes of all kinds of ink-absorbing recording medium, either aluminum oxide (Al₂O₃) or aluminum oxide solutions (Al(OH)₃) can be used and all kinds of base materials are suitable. Moreover, for aluminum oxide or aluminum oxide solutions, in order to insure the ink-absorbing ability, poriferous base materials are preferable after applying the aluminum oxide and the drying processes. For example, α-aluminum such as AKP15 (made by Sumitomo) is adapted to aluminum oxide (Al₂O₃) used for ink-jet papers which use dye ink. Crystalline boehmite (A100H) such as DISPERAL HP 14 (made by Sasol) can be used for aluminum oxide solutions. For DISPERAL HP 14, the particle status that the surface area can be measured by BET adhesion method is preferably 150-170 m²/g, and the pore volume total is preferably 0.9∼1.2 ml/g.

There are many material choices for membrane layers, like aluminum oxide, aluminum oxide or solutions which contain water-dispersibility property and aqueous colloids. Acetic acid and nitric acid are used to adjust pH values to 4-5 due to their fine dispersing ability.

As mentioned above, in the past, non-ion or anionic polyvinyl alcohol is used as membrane layer liquid, however, in the present invention, membrane layer liquid which does not contain polyvinyl alcohol is used to form the membrane layer. Subsequently, the cationic polyvinyl alcohol is added into the membrane layer by smearing cationic polyvinyl alcohol solutions thereon. The cationic polyvinyl alcohol can be smeared on the membrane layer either after or during the drying process.

There are other processes to generate gelation on the membrane layer of the base material. C506 (made by Kuraray) and K210 (made by Gohsenol) can be used as cationic polyvinyl alcohol. High concentration polyvinyl alcohol can prevent the membrane layer from fading, and the viscosity of high concentration polyvinyl alcohol will be very low when it is dissolved in water. Besides, low molecular weight is preferred because the polyvinyl alcohol has to permeate deeply into the membrane layer. Preferably, the molecular weight should be less than 2000g.

With regard to the coater, Comma Coater and Kiss Coater can be used, and the Comma Coater is preferable. The ink-absorbing recording medium of the present invention can be used in ink-jet printers and flexography printing. The fabricating process of the ink-absorbing recording medium described in the first embodiment uses paper as the base material and casts the membrane layer which is applied with cationic polyvinyl alcohol to make a glossy ink-absorbing recording medium. The casting process discussed here is to press the moistened membrane layer onto the heated metal surface to be dried. During the casting process, dried or semi-dried membrane layer which is made of aluminum oxide is applied with cationic polyvinyl alcohol and then cast when it is moistened. Compared with those processes during which casting process is carried out when the membrane layer is still wet, the present process can make the membrane layer dry more quickly, and easier to be detached from the metal surface. Besides, one of the purposes for casting is to make the ink-absorbing recording medium glossy.

The following is the second embodiment for manufacturing the ink-absorbing recording medium according to the present invention. In the second embodiment, cationic aluminum oxide, cationic aluminum oxide solution, or cationic polyvinyl alcohol is applied to the base material to form the membrane layer. Therefore, in the second embodiment, cationic polyvinyl alcohol is used to form the membrane layer so as to fabricate the ink-absorbing recording medium with good ink-absorbing and weather-enduring abilities.

Gelation phenomenon is easy to happen when the non-ion or anionic polyvinyl alcohol or the aluminum oxide is selected as the membrane liquid. However, in the present invention, gelation seldom happens because cationic polyvinyl alcohol and aluminum oxide repel to each other so that the solution is spread uniformly.

In comparison with the first embodiment, in the second embodiment, the polyvinyl alcohol is mixed in the membrane liquid in advance, so that the membrane can be formed easier and is more uniform.

Fig. 3(b) is a diagram showing the inferential status of aluminum oxide particles and cationic polyvinyl alcohol in the membrane layer of the ink-absorbing recording medium fabricated by the above-mentioned process of the second embodiment. Those aluminum oxide particles which are covered by the cationic polyvinyl alcohol adhere to each other after the aluminum oxide particles and the cationic polyvinyl alcohol are mixed. Compared with Fig. 3(a), the spaces between the aluminum particles can absorb dye more easily and maybe get exposed in ozone. However, in comparison with the prior art illustrated in Fig. 3(c), in the present invention cationic polyvinyl alcohol is used so that active points can be covered and therefore, the ink-absorbing recording medium is endurable to weather.

According to the second preferred embodiment of the present invention, the steps of making an ink-absorbing recording medium are: forming a membrane layer on a paper base material; drying or semi-drying the membrane layer; applying water to the dried or semi-dried membrane layer; and then casting the water-applied membrane layer to make a glossy ink-absorbing recording medium.

A membrane layer containing cationic aluminum oxide or cationic aluminum oxide solutions and cationic polyvinyl alcohol is formed on the base material of the ink-absorbing recording medium. Here, either cationic aluminum oxide or cationic aluminum oxide solution can be chosen, and cationic polyvinyl alcohol with a lower molecular weight is preferred. A excellent ink-absorbing recording medium with good ink-absorbing and weather-enduring abilities is available because cationic polyvinyl alcohol is used as the adhesive of the membrane layer. Preferably, the weight percent of the cationic aluminum oxide or the cationic aluminum oxide solution is 70%∼95% and that of the cationic polyvinyl alcohol is 30%∼5%.

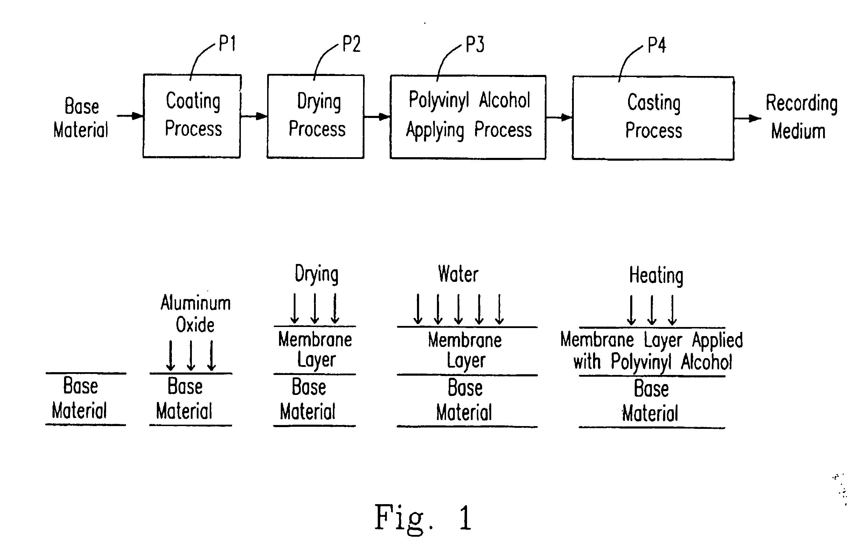

The present invention will now be described more specifically with reference to the following embodiments. Fig. 1 shows the flow chart representing the first embodiment of the method of manufacturing the ink-absorbing recording medium. In the coating process P1, a coater is used to apply the membrane liquid to the surface of the paper base material and hence a membrane layer is formed thereon. A Comma Coater is used as the coater and the colloidal quasi-boehmite dispersed in the water is used as the membrane liquid in this embodiment. Preferably, the weight percent of the membrane liquid is 23%. Acetic acid is added to keep the pH value of the membrane liquid at 4.0 so as to maintain dispersibility and colloidal status.

The drying process P2 is to dry the membrane layer formed in the coating process P1.

In polyvinyl alcohol applying process P3, through a Comma Coater, the cationic polyvinyl alcohol solution is applied to the surface of the membrane layer after it is dried in the drying process P2. The applied solution is permeated into the membrane layer so that the cationic polyvinyl alcohol solution permeates accordingly.

In the casting process P₄, a casting process is performed by a heated casting cylinder on the surface of the membrane layer applied by the cationic polyvinyl alcohol solution. Therefore, the membrane layer of the paper base material where the cationic polyvinyl alcohol solution permeates, i.e., the part which contains quasi-boehmite and cationic polyvinyl alcohol solution, is dried after this process.

All kinds of equipments may be used as the cylinder. In this embodiment, the cylinder is plated with chromium. The cylinder is heated by steam, and the membrane layer which is applied with cationic polyvinyl alcohol solution is dried after it is pressed by the cylinder.

Through the above-mentioned steps, the final product made by the casting process P4 is the ink-absorbing recording medium. The surface density of the solid part of the product which is made of aluminum oxide solution and polyvinyl alcohol is 25 g/m². The ink-absorbing recording medium achieved in this embodiment has the membrane layer on the paper base material wherein the membrane layer contains quasi-boehmite and cationic polyvinyl alcohol.

Further, here is a comparison of the qualities of the ink-absorbing recording medium made by the present method and the conventional method. The membrane liquid of the ink-absorbing recording medium used in the conventional method utilizes DISPERAL HP 14 of quasi-boehmite as aluminum oxide or the aluminum oxide solution, JM-26 as non-ion or anionic polyvinyl alcohol, and the rest of the liquid is water. DISPERAL HP 14 is made by Sosal and JM-26 is made by Japan Vam and Poval. The weight percent of DISPERAL HP 14 is 23% and the weight percent of JM-26 is 2.3%. In the ink-absorbing recording medium made by the conventional method, the surface densities of dried DISPERAL HP 14 and JM-26 on the paper base material are 25 g/m².

Moreover, in the ink-absorbing recording medium made by the conventional method, the weight percent of aluminum oxide to the dried solid part of the polyvinyl alcohol is 10%, but that of the present invention is 20%.

The following is an example to describe the ink-absorbing ability of the ink-absorbing recording medium. First, we use Photoshop to generate the highest concentration colors of red, blue and green and then print them by Canon printer BJF870. As a result, for the conventional ink-absorbing recording medium, the drops of ink gather together and the color changes before they are absorbed by the recording medium; however, for the ink-absorbing recording medium provided in the present invention, the color is not changed and the ink is absorbed uniformly by the recording medium.

The following is an example to describe the ozone-enduring ability of the ink-absorbing recording medium. During May and June, in which the intensity of ultraviolet rays is very high and the oxidation caused by ozone is the most serious, the conventional and present ink-absorbing recording mediums are put indoors with normal light in Tokyo. After one month, attenuation rates of the colors are recorded. The average of the attenuation rates of red, blue, green, and black is calculated and denoted by the symbol ΔE. It is found thatΔE of the conventional ink-absorbing recording medium is 31%, but that of the present one is only 12%. This means that the present ink-absorbing recording medium is more endurable to ozone-worsened weather. Further, there is little difference between the conventional and the present ink-absorbing recording mediums in the respect of the light-worsened weather-enduring ability.

Next, Gohsenol K210 (made by Japan Chemical Company, Ltd) and C506 (made by Kuraray) are both used as cationic polyvinyl alcohol in the ink-absorbing recording medium, and the results are compared. We use Photoshop to generate the highest concentration colors of red, blue and green and then print them on these two recording mediums respectively by Canon printer BJF870. As a result, for Gohsenol K210, the concentrations of red, blue, green and black colors are 2.2, 2.4, 2.2, and 2.3, respectively, and those for C506 are 1.9, 2.2, 2.1, and 2.1, respectively. It is obvious that the concentration of color of recording medium which employs Gohsenol K210 is higher than that of the recording medium which employs C506. It is because that Gohsenol K210 is more basic than C506, and C506 is less endurable.

Specifically, the alkali of Gohsenol K210 is 85.5∼88.0 (mol%), and that of C506 is 74.0∼80.0 (mol %). Therefore, cationic high alkali is preferred because it is more easily dissolved in water. Preferably, the alkali of cationic polyvinyl alcohol is more than 90 (mol %). Moreover, low condensation is preferred in the cationic polyvinyl alcohol because it has good weather-enduring ability and high color concentration.

The following is the description of the effects of the ink-absorbing recording medium in the present invention. By following the method of manufacturing the ink-absorbing recording medium in the present invention, an ink-absorbing recording medium with high quality can be made. The ink-absorbing recording medium of the present invention has good ink-absorbing ability. When ink is used for printing, it is endurable to ozone or NOₓ. When an anionic dye is used, the adhesion between the dye and the polyvinyl alcohol is very good because the membrane layer of the ink-absorbing recording medium in the present invention utilizes cationic polyvinyl alcohol. High quality printing papers are provided by using the method of manufacturing ink-absorbing recording medium presented in the present invention. The papers are suitable for various kinds of printers which use ink, carbon powder or dyes. The ink-absorbing recording medium presented in the present invention had good adhesion ability for carbon powder and is also suitable for photographic paper in digital photography fields. Moreover, the ink-absorbing recording medium is suitable for flexography printing as well because the ink on plastic plates dries very quickly.

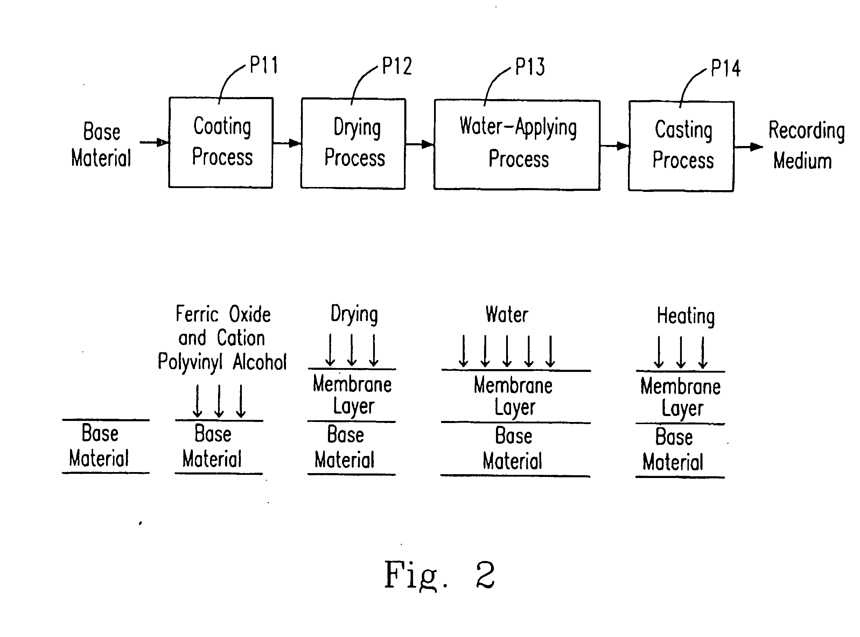

Next, Fig. 2 illustrates the whole processes for manufacturing the ink-absorbing recording medium according to the second embodiment of the present invention. In the second embodiment of the present invention, paper is used as the base material, and is treated under coating process P11, drying process P12, water applying process P13, and casting process P14. Then the product obtained from the above processes is ink-absorbing recording medium rolled on the cylinder.

In the coating process P11, a coater is used to apply the membrane liquid to the surface of the paper base material and hence a membrane layer is formed thereon. A Comma Coater is used as the coater and quasi-boehmite and cationic polyvinyl alcohol solutions are used as the membrane liquid in this embodiment. Some acetic acid or nitric acid is added to maintain the PH value around 4 to 5. Preferably, DISPERAL HP 14 of the quasi-boehmite is dissolved in the water, and the weight percent is 23%. And GohsenolK210 of the polyvinyl alcohol made by the Nippon Synthetic Chemical Industry is dissolved in the water to make a solution of weight percent of 13%. Moreover, in the ink-absorbing recording medium of this embodiment, the weight percent of aluminum oxide to the dried solid part of the polyvinyl alcohol is 15%.

The drying process P12 is to dry the membrane layer formed in the coating process P11 from the surface. A semi-drying process can also be adapted in the drying process P12.

The water applying process P13 is to apply water to the surface of the membrane layer dried in the drying process P12.

In the casting process P14, a casting process is performed by a heated casting cylinder on the surface of the membrane layer which is applied by water. A glossy ink-absorbing recording medium is accomplished by the casting process P14. The product (paper base material with membrane layer) of casting process P14 is the ink-absorbing recording medium. The ink-absorbing recording medium achieved in this embodiment has the membrane layer on the paper base material wherein the membrane layer contains quasi-boehmite and cationic polyvinyl alcohol. In comparison with the conventional previous ink-absorbing recording medium, the ink-absorbing recording medium achieved in this embodiment has better ink-absorbing ability and is more endurable to weather.

Fig. 3(a) is a diagram showing the inferential status of aluminum oxide particles and cationic polyvinyl alcohol in the membrane layer of the ink-absorbing recording medium fabricated by the above-mentioned process. Fig. 3(b) is a diagram showing the inferential status of aluminum oxide particles and cationic polyvinyl alcohol in the membrane layer of the ink-absorbing recording medium fabricated by the above-mentioned process of the second embodiment. Those aluminum oxide particles which are covered by the cationic polyvinyl alcohol adhere to each other after the aluminum oxide particles and the cationic polyvinyl alcohol are mixed. Compared with Fig. 3(a), the spaces between the aluminum particles can absorb dye more easily and maybe get exposed in ozone. However, in comparison with the prior art illustrated in Fig. 3(c), in the present invention cationic polyvinyl alcohol is used so that active points can be covered and therefore, the ink-absorbing recording medium is endurable to weather.

## Claims

1. A method of manufacturing an ink-absorbing recording medium, **characterized by** the following steps:
a) providing a liquid, which is formed by one of a cationic aluminum oxide and a dispersing solution or a colloid solution which mainly comprises a cationic aluminum oxide solution and applying said liquid on a base material to form a membrane layer;
b) drying the membrane layer; and
c) applying a cationic polyvinyl alcohol solution to the membrane layer, wherein the cationic polyvinyl alcohol and cationic aluminium oxide repel to each other.

2. The method as claimed in claim 1, **characterized in that** the cationic polyvinyl alcohol solution is a high alkali cationic polyvinyl alcohol solution.

3. The method as claimed in claim 2, **characterized in that** the cationic polyvinyl alcohol solution has an alkali of 85.5 to 88.0 mol%.

4. The method as claimed in claim 2, **characterized in that** the cationic polyvinyl alcohol solution has an alkali of 74.0 to 80.0 mol%.

5. The method as claimed in claim 2, **characterized in that** the cationic polyvinyl alcohol solution has an alkali of more than 90 mol%.

6. The method as claimed in claim 1, **characterized in that** the drying step is performed by one of a drying process (P2) and a semi-drying process.

7. The method as claimed in claim 1, **characterized in that** the base material is a paper material, and the membrane layer applied with the cationic polyvinyl alcohol is cast through a casting process (P4) so as to manufacture a glossy ink-absorbing recording medium.

8. A method of manufacturing an ink-absorbing recording medium **characterized by** the following steps:
a) applying a liquid, which is formed by one of a cationic aluminum oxide and a dispersing solution or a colloid solution which mainly comprises a cationic aluminum oxide solution and then a cationic polyvinyl alcohol solution on a base material to form a membrane layer, wherein the cationic polyvinyl alcohol and cationic aluminium oxide repel to each other,
b) drying the membrane layer.

9. The method as claimed in claim 8, **characterized in that** the cationic aluminum oxide and the cationic aluminum oxide solution both have a concentration by weight of 70%∼95% and the corresponding cationic polyvinyl alcohol has a concentration by weight of 5%∼30%.

10. The method as claimed in claim 8, **characterized in that** the base material is a paper material, the membrane layer is dried by a drying process (P12), and the water-applied membrane layer is cast through a casting process (P14) for manufacturing a glossy ink-absorbing recording medium.

11. The method as claimed in claim 10, **characterized in that** the drying process (P12) is one of a drying process (P12) and a semi-drying process.

12. The method as claimed in claim 8, **characterized in that** the cationic polyvinyl alcohol solution is a high alkali cationic polyvinyl alcohol solution.

13. The method as claimed in claim 8, **characterized in that** the cationic polyvinyl alcohol solution has an alkali of 85.5 to 88.0 mol%.

14. The method as claimed in claim 8, **characterized in that** the cationic polyvinyl alcohol solution has an alkali of 74.0 to 80.0 mol%.

15. The method as claimed in claim 8, **characterized in that** the cationic polyvinyl alcohol solution has an alkali of more than 90 mol%.

16. An ink-absorbing recording medium having a membrane layer, **characterized in that** the membrane layer comprises a cationic aluminum oxide and a cationic polyvinyl alcohol, wherein the cationic polyvinyl alcohol and cationic aluminium oxide repel to each other so that the cationic aluminum oxide is covered by the cationic polyvinyl alcohol.

17. The ink-absorbing recording medium as claimed in claim 16, **characterized in that** the cationic aluminum oxide is completely covered by the cationic polyvinyl alcohol.

## Patentansprüche

1. Verfahren zur Herstellung eines tintenaufnehmenden Aufzeichnungsmediums, **gekennzeichnet durch** die folgenden Schritte:
a) Bereitstellen einer Flüssigkeit, die **durch** ein kationisches Aluminiumoxid und eine Dispergierlösung oder eine Kolloidlösung, die hauptsächlich eine Lösung von kationischem Aluminiumoxid umfasst, gebildet wird, und Aufbringen der Flüssigkeit auf ein Basismaterial zur Bildung einer Membranschicht;
b) Trocknen der Membranschicht und
c) Aufbringen einer Lösung von kationischem Polyvinylalkohol auf die Membranschicht, wobei der kationische Polyvinylalkohol und das kationische Aluminiumoxid einander abstoßen.

2. Verfahren nach Anspruch 1, **dadurch gekennzeichnet, dass** es sich bei der Lösung von kationischem Polyvinylalkohol um eine alkalireiche Lösung von kationischem Polyvinylalkohol handelt.

3. Verfahren nach Anspruch 2, **dadurch gekennzeichnet, dass** die Lösung von kationischem Polyvinylalkohol einen Alkaligehalt von 85,5 bis 88,0 Mol-% aufweist.

4. Verfahren nach Anspruch 2, **dadurch gekennzeichnet, dass** die Lösung von kationischem Polyvinylalkohol einen Alkaligehalt von 74,0 bis 80,0 Mol-% aufweist.

5. Verfahren nach Anspruch 2, **dadurch gekennzeichnet, dass** die Lösung von kationischem Polyvinylalkohol einen Alkaligehalt von mehr als 90 Mol-% aufweist.

6. Verfahren nach Anspruch 1, **dadurch gekennzeichnet, dass** der Trocknungsschritt durch einen Trocknungsprozess (P2) oder einen Teiltrocknungsprozess durchgeführt wird.

7. Verfahren nach Anspruch 1, **dadurch gekennzeichnet, dass** es sich bei dem Basismaterial um ein Papiermaterial handelt und die aufgebrachte Membranschicht mit dem kationischen Polyvinylalkohol durch einen Gießprozess (P4) gegossen wird, um ein glänzendes tintenaufnehmendes Aufzeichnungsmedium herzustellen.

8. Verfahren zur Herstellung eines tintenaufnehmenden Aufzeichnungsmediums, **gekennzeichnet durch** die folgenden Schritte:
a) Aufbringen einer Flüssigkeit, die **durch** ein kationisches Aluminiumoxid und eine Dispergierlösung oder eine Kolloidlösung, die hauptsächlich eine Lösung von kationischem Aluminiumoxid umfasst, gebildet wird, und dann einer Lösung von kationischem Polyvinylalkohol auf ein Basismaterial zur Bildung einer Membranschicht, wobei der kationische Polyvinylalkohol und das kationische Aluminiumoxid einander abstoßen;
b) Trocknen der Membranschicht.

9. Verfahren nach Anspruch 8, **dadurch gekennzeichnet, dass** das kationische Aluminiumoxid und die Lösung von kationischem Aluminiumoxid beide eine Konzentration von 70-95 Gew.-% aufweisen und der entsprechende kationische Polyvinylalkohol eine Konzentration von 5-30 Gew.-% aufweist.

10. Verfahren nach Anspruch 8, **dadurch gekennzeichnet, dass** es sich bei dem Basismaterial um ein Papiermaterial handelt, die Membranschicht durch einen Trocknungsprozess (P12) getrocknet wird und die wässrig aufgebrachte Membranschicht durch einen Gießprozess (P14) zur Herstellung eines glänzenden tintenaufnehmenden Aufzeichnungsmediums gegossen wird.

11. Verfahren nach Anspruch 10, **dadurch gekennzeichnet, dass** es sich bei dem Trocknungsprozess (P12) um einen Trocknungsprozess (P12) oder einen Teiltrocknungsprozess handelt.

12. Verfahren nach Anspruch 8, **dadurch gekennzeichnet, dass** es sich bei der Lösung von kationischem Polyvinylalkohol um eine alkalireiche Lösung von kationischem Polyvinylalkohol handelt.

13. Verfahren nach Anspruch 8, **dadurch gekennzeichnet, dass** die Lösung von kationischem Polyvinylalkohol einen Alkaligehalt von 85,5 bis 88,0 Mol-% aufweist.

14. Verfahren nach Anspruch 8, **dadurch gekennzeichnet, dass** die Lösung von kationischem Polyvinylalkohol einen Alkaligehalt von 74,0 bis 80,0 Mol-% aufweist.

15. Verfahren nach Anspruch 8, **dadurch gekennzeichnet, dass** die Lösung von kationischem Polyvinylalkohol einen Alkaligehalt von mehr als 90 Mol-% aufweist.

16. Tintenaufnehmendes Aufzeichnungsmedium mit einer Membranschicht, **dadurch gekennzeichnet, dass** die Membranschicht ein kationisches Aluminiumoxid und einen kationischen Polyvinylalkohol umfasst, wobei der kationische Polyvinylalkohol und das kationische Aluminiumoxid einander abstoßen, so dass das kationische Aluminiumoxid mit dem kationischen Polyvinylalkohol bedeckt ist.

17. Tintenaufnehmendes Aufzeichnungsmedium nach Anspruch 16, **dadurch gekennzeichnet, dass** das kationische Aluminiumoxid vollständig mit dem kationischen Polyvinylalkohol bedeckt ist.

## Revendications

1. Procédé de fabrication d'un support d'enregistrement absorbant l'encre, **caractérisé par** les étapes suivantes :
a) la fourniture d'un liquide, qui est constitué par l'un parmi un oxyde d'aluminium cationique et une solution de dispersion ou une solution colloïdale qui comprend principalement une solution d'oxyde d'aluminium cationique et l'application dudit liquide sur un matériau de base pour former une couche membranaire ;
b) le séchage de la couche membranaire ; et
c) l'application d'une solution d'alcool polyvinylique cationique sur la couche membranaire, l'alcool polyvinylique cationique et l'oxyde d'aluminium cationique se repoussant l'un l'autre.

2. Procédé selon la revendication 1, **caractérisé en ce que** la solution d'alcool polyvinylique cationique est une solution d'alcool polyvinylique cationique à teneur en alcalis élevée.

3. Procédé selon la revendication 2, **caractérisé en ce que** la solution d'alcool polyvinylique cationique a une teneur en alcalis de 85,5 à 88,0 % en mole.

4. Procédé selon la revendication 2, **caractérisé en ce que** la solution d'alcool polyvinylique cationique a une teneur en alcalis de 74,0 à 80,0 % en mole.

5. Procédé selon la revendication 2, **caractérisé en ce que** la solution d'alcool polyvinylique cationique a une teneur en alcalis supérieure à 90 % en mole.

6. Procédé selon la revendication 1, **caractérisé en ce que** l'étape de séchage est effectuée par l'un parmi un procédé de séchage (P2) et un procédé de semi-séchage.

7. Procédé selon la revendication 1, **caractérisé en ce que** le matériau de base est un matériau en papier et la couche membranaire sur laquelle est appliqué l'alcool polyvinylique cationique est coulée par un procédé de coulage (P4) afin de fabriquer un support d'enregistrement absorbant l'encre brillant.

8. Procédé de fabrication d'un support d'enregistrement absorbant l'encre **caractérisé par** les étapes suivantes consistant à :
a) l'application d'un liquide, qui est constitué par l'un parmi un oxyde d'aluminium cationique et une solution de dispersion ou une solution colloïdale qui comprend principalement une solution d'oxyde d'aluminium cationique et ensuite d'une solution d'alcool polyvinylique cationique sur le matériau de base pour former une couche membranaire, l'alcool polyvinylique cationique et l'oxyde d'aluminium cationique se repoussant l'un l'autre,
b) le séchage de la couche membranaire.

9. Procédé selon la revendication 8, **caractérisé en ce que** l'oxyde d'aluminium cationique et la solution d'oxyde d'aluminium cationique ont tous deux une concentration en poids de 70 %-95 % et l'alcool polyvinylique cationique correspondant a une concentration en poids de 5 %-30 %.

10. Procédé selon la revendication 8, **caractérisé en ce que** le matériau de base est un matériau en papier, la couche membranaire est séchée par un procédé de séchage (P12) et la couche membranaire appliquée avec de l'eau est coulée par un procédé de coulage (P14) pour fabriquer un support d'enregistrement absorbant l'encre brillant.

11. Procédé selon la revendication 10, **caractérisé en ce que** le procédé de séchage (P12) est l'un parmi un procédé de séchage (P12) et un procédé de semi-séchage.

12. Procédé selon la revendication 8, **caractérisé en ce que** la solution d'alcool polyvinylique cationique est une solution d'alcool polyvinylique cationique à teneur en alcalis élevée.

13. Procédé selon la revendication 8, **caractérisé en ce que** la solution d'alcool polyvinylique cationique a une teneur en alcalis de 85,5 à 88,0 % en mole.

14. Procédé selon la revendication 8, **caractérisé en ce que** la solution d'alcool polyvinylique cationique a une teneur en alcalis de 74,0 à 80,0 % en mole.

15. Procédé selon la revendication 8, **caractérisé en ce que** la solution d'alcool polyvinylique cationique a une teneur en alcalis supérieure à 90 % en mole.

16. Support d'enregistrement absorbant l'encre ayant une couche membranaire, **caractérisé en ce que** la couche membranaire comprend un oxyde d'aluminium cationique et un alcool polyvinylique cationique, l'alcool polyvinylique cationique et l'oxyde d'aluminium cationique se repoussant l'un l'autre de sorte que l'oxyde d'aluminium cationique est recouvert par l'alcool polyvinylique cationique.

17. Support d'enregistrement absorbant l'encre selon la revendication 16, **caractérisé en ce que** l'oxyde d'aluminium cationique est complètement recouvert par l'alcool polyvinylique cationique.
